(19) Europäisches Patentamt
European Patent Office
Office européen des brevets

(11) Numéro de publication : **0 106 860**
**B1**

(12) # FASCICULE DE BREVET EUROPÉEN

(45) Date de publication du fascicule du brevet : **20.01.88**

(51) Int. Cl.$^4$ : **C 07 D405/06**, A 61 K 31/445

(21) Numéro de dépôt : **83901206.9**

(22) Date de dépôt : **21.04.83**

(86) Numéro de dépôt international : **PCT/FR 83/00074**

(87) Numéro de publication internationale : **WO/8303829 (10.11.83 Gazette 83/26)**

(54) **NOUVELLES CARBOXAMIDOGUANIDINES, LEUR PROCEDE D'OBTENTION ET LES COMPOSITIONS PHARMACEUTIQUES EN RENFERMANT.**

(30) Priorité : **21.04.82 FR 8206832**

(43) Date de publication de la demande : **02.05.84 Bulletin 84/18**

(45) Mention de la délivrance du brevet : **20.01.88 Bulletin 88/03**

(84) Etats contractants désignés : **AT BE CH DE FR GB LI LU NL SE**

(56) Documents cités : **EP-A- 0 004 358**

(73) Titulaire : **Bouchara, Emile, Dr.
Le Parc Royal 16, rue du Parc Royal
F-75003 Paris (FR)**

(72) Inventeur : **CORNU, Pierre Jean
100, avenue Kléber
F-75116 Paris (FR)**
Inventeur : **PERRIN, Claude
5, rue de l'Avenir
F-91400 Orsay (FR)**
Inventeur : **DUMAITRE, Bernard
24, rue du Chemin Vert
F-93000 Bobigny (FR)**
Inventeur : **STREICHENBERGER, Gilles
30, boulevard du Château
F-92200 Neuilly-sur-Seine (FR)**

(74) Mandataire : **Burtin, Jean-François
5 bis, rue Parmentier
F-92200 Neuilly s/Seine (FR)**

EP 0 106 860 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention a pour objet de nouvelles guanidines substituées, leur procédé d'obtention ainsi que les compositions pharmaceutiques en contenant.

L'invention a plus particulièrement pour objet des guanidines trisubstituées liées à une pipéridine.

Elle a spécifiquement pour objet les carboxamidoguanidines répondant à la formule générale I

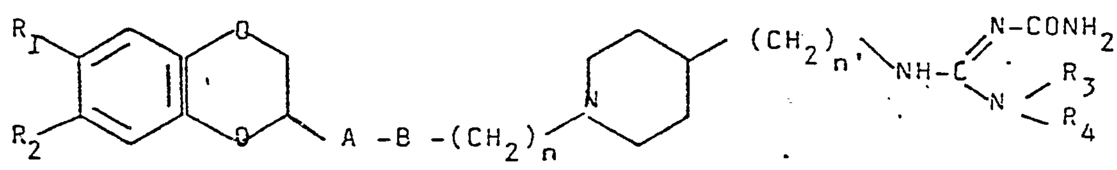

(I)

dans laquelle

$R_1$ et $R_2$, distinctement l'un de l'autre, représentent de l'hydrogène, un halogène, un radical alcoyle ayant de 1 à 6 atomes de Carbone, un radical alcoxy inférieur dont la partie alcoyle comporte de 1 à 6 atomes de Carbone ou un radical trifluorométhyle.

$R_3$ représente un radical alcoyle inférieur ayant de 1 à 6 atomes de Carbone, un radical cyclopropyle ou un radical allyle.

$R_4$ représente de l'hydrogène, un radical alcoyle inférieur ayant de 1 à 6 atomes de Carbone ou un reste acyle dérivé d'un acide carboxylique ayant de 1 à 12 atomes de Carbone,

ou bien $R_3$ et $R_4$ forment ensemble le reste alcoylène d'un hétérocycle azoté ayant de 3 à 7 maillons pouvant comporter un autre hétéroatome, choisi dans le groupe constitué par l'azétidine, l'aziridine, la pyrolidine, la pipéridine, l'hexahydropyrimidine, la tétrahydrothiazine, l'hexaméthylène imine, l'heptaméthylène imine, la morpholine, la pipérazine, une alcoylpipérazine, une (hydroxyalcoyl) pipérazine et une (acyloxyalcoyl) pipérazine.

A représente une liaison simple ou —CH2— et B représente —CHOH— ou —C = 0 ou A représente —CHOH— ou C = 0 et B représente une liaison simple ou —CH2—.

L'invention se rapporte aussi aux sels d'addition des composés de formule générale I avec un acide minéral ou organique, de préférence un acide thérapeutiquement compatible.

La molécule comporte au moins un atome de carbone asymétrique et de ce fait après dédoublement on peut obtenir les isomères lévogyres et dextrogyres des composés de formule générale I.

Par ailleurs, compte tenu de la rigidité de la liaison —C = N— le groupe carboxamide peut se trouver d'un côté ou de l'autre du plan déterminé par cette liaison. Il en résulte une possibilité d'existence d'isomérie syn et anti. Ces isomères peuvent être séparés par des méthodes physiques.

L'invention s'étend donc aussi aux isomères optiquement actifs des composés de formule générale I et aux isomères syn et anti des composés de formule générale I.

La formule générale I représente une des structures possibles des carboxamidoguanidines. En milieu acide l'un quelconque des azotes de la fonction guanidine peut être protoné. Il en résulte que les composés selon l'invention peuvent exister sous les deux formes tautomères imino-carboxamido-amine et amino-carboxamido-imine.

Parmi les sels d'addition des composés de formule générale I, on pourra citer plus particulièrement les chlorhydrates, bromhydrates, sulfates, nitrates, phosphates, thiosulfates, les formiates, acétates, maléates, fumarates, benzoates, dichloro 2,6-benzoates, citrates, tartrates, (méthoxy salicylates), 3,4,5-triméthoxy benzoates, les vanillinates, les O-carbéthoxy syringoates, les naphtoates, les benzène sulfonates, les méthane sulfonates, les iséthionates, les nicotinates, les isonicotinates, les embonates et les glucose-phosphates.

Pour autant que l'invention soit concernée, un radical alcoyle inférieur est une chaîne hydrocarbonée ayant de 1 à 6 atomes de Carbone en chaîne droite ou ramifiée, comme par exemple le méthyle, l'éthyle, l'isopropyle, le secbutyle, le terbutyle, le pentyle, le néopentyle et le n-hexyle.

Un radical alcoxy inférieur a de 1 à 6 atomes de Carbone dans la chaîne alcoyle qui peut être droite ou ramifiée comme un méthoxy, un éthoxy, un isopropoxy, un terbutoxy ou un pentyloxy.

Un radical acyle est dérivé d'un acide organique carboxylique ayant de 1 à 12 atomes de Carbone, comme par exemple un acide alcoyl carboxylique, un acide arylcarboxylique, un acide arylalcoyl carboxylique, un acide cycloalcoyl-carboxylique ou un acide hétéroaryl carboxylique. On pourra citer à cet égard un acétyle, un butyryle, un benzoyle, un 3,4,5-triméthoxybenzoyle, un cyclopropylcarbonyle ou un nicotinoyle.

La signification des paramètres n et n' est importante et joue un rôle significatif dans les propriétés pharmacologiques des composés de formule générale I. L'intensité ou la durée d'action des composés, selon l'invention, peut être modulée en modifiant la longueur de la chaîne carbonée de l'une ou l'autre partie de la molécule.

Lorsque R3 et R4 forment ensemble la chaîne alcoylène d'un hétérocycle azoté, ils représentent le reste d'un cycle aziridine, azétidine, pyrolidine, pipéridine, hexahydropyrimidine, tétrahydrothiazine,

hexaméthylène imine, heptaméthylène imine morpholine, pipérazine, alcoylpipérazine, hydroxyalcoyl pipérazine ou acyloxyalcoyl pipérazine.

Parmi les composés de l'invention on citera plus particulièrement :

les composés de formule IA

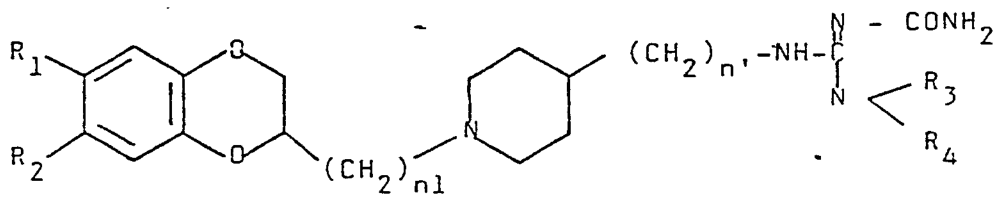

(IA)

dans laquelle n1 représente un nombre entier variant de 0 à 3 et les substituants R1, R2, R3, R4 et n'ont les significations fournies précédemment, ainsi que les sels d'addition de ces composés avec un acide minéral ou organique,

les composés de formule IB

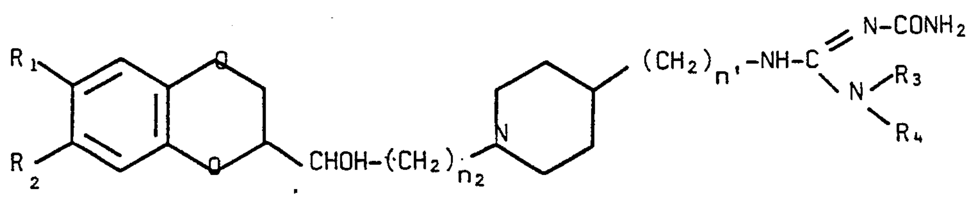

(IB)

dans laquelle n2 représente un nombre entier variant de 0 à 2 et les substituants R1, R2, R3, R4 et n' ont les définitions précédentes, ainsi que les sels d'addition de ces composés avec un acide minéral ou organique,

les composés de formule IC

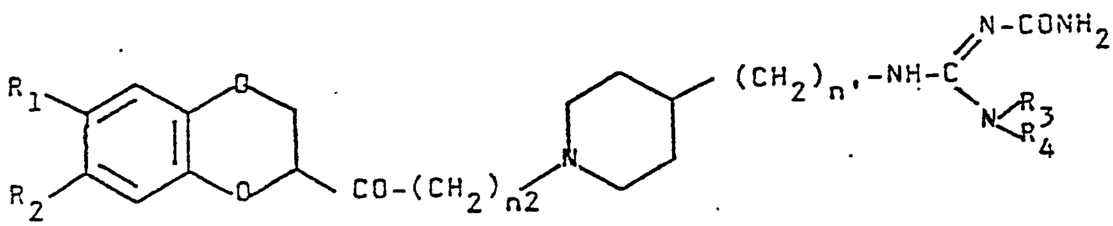

IC

dans laquelle $R_1$, $R_2$, $R_3$, $R_4$, n' et $n_2$ ont les significations fournies précédemment ainsi que leurs sels d'addition avec un acide minéral ou organique et spécifiquement :

la 1-[2,3 dihydro [4H] (1,4-benzodioxin-2yl) méthyl] 4-(N-carboxamido N'-méthyl guanidinyl) pipéridine

la 1-[2,3-dihydro [4H] (1,4-benzodioxin-2yl) méthyl] 4-[(N-carboxamido N'-méthyl] guanidinyl) méthyl pipéridine

la 1-[2,3-dihydro [4H] (1,4-benzodioxin-2yl) méthyl] 4-[(N-carboxamido N'-allyl) guanidinyl] pipéridine

la 1-[(2,3-dihydro [4H] 1,4-benzodioxin-2yl) méthyl] 4-[(N-carboxamido N'-allyl guanidinyl) méthyl] pipéridine

la 1-[(2,3-dihydro [4H] 1,4-benzodioxin-2yl) méthyl] 4-[(N-carboxamido N'-triméthyl propyl guanidinyl) méthyl] pipéridine

la 1-[(2,3-dihydro [4H] 1,4-benzodioxin-2yl) méthyl] 4-(N-carboxamido N'-cyclopropyl guanidinyl) méthyl pipéridine

1a 1-[(2,3-dihydro [4H] 1,4-benzodioxin-2yl) méthyl] 4-[(N-carboxamido N'-morpholino guanidinyl) méthyl] pipéridine.

Les composés selon l'invention se distinguent par leurs propriétés pharmacologiques intéressantes et notamment par leurs propriétés anti-hypertensives associées à une action faiblement sédative sur le Système Nerveux Central. Du fait de leur haut niveau d'activité, les composés de formule générale I ou leurs sels d'addition trouvent un emploi en thérapeutique humaine ou animale comme principes actifs de médicaments destinés à combattre ou à réduire les effets de la maladie hypertensive.

Ils se différencient des composés du type Imidazolinone décrits dans la demande de brevet européen 4358 non seulement par la structure chimique mais par un ensemble de propriétés pharmacologiques. Les composés décrits antérieurement sont des médicaments du Système Nerveux Central agissant soit comme stimulants ou comme anti-dépresseurs. Les composés, selon l'invention, ne manifestent au contraire que des propriétés très limitées sur le Système Nerveux Central.

Les composés selon l'invention sont utilisés sous forme de compositions pharmaceutiques destinées

à l'administration par voie parentérale, buccale, rectale ou sublinguale.

Les compositions pharmaceutiques renferment, comme principe actif, au moins un composé de formule générale I ou un de ses sels d'addition avec un acide minéral ou organique, en association ou en mélange avec un excipient ou un véhicule inerte pharmaceutiquement acceptable.

On pourra citer comme formes d'administrations préférées les comprimés nus ou enrobés, les capsules, les gélules, les dragées, les comprimés à noyaux multiples, les gouttes, les solutions ou suspensions buvables ; les solutés ou suspensions injectables répartis en ampoules, en flacons multidoses ou en seringues auto-injectables ; les suppositoires ; les comprimés sublinguaux.

Les compositions pharmaceutiques selon l'invention peuvent en outre renfermer un ou d'autres principes actifs d'action similaire, complémentaire ou synergique. On pourra ainsi ajouter un diurétique du type thiazidique ou du type triaminoptéridine ; ou un agent bêta bloqueur comme le propranolol, le pindolol ou l'aténolol.

La posologie journalière peut varier entre des limites larges en fonction de l'indication thérapeutique, de la voie d'administration, de l'âge du malade et de l'ancienneté de la maladie hypertensive. En règle générale, chez l'adulte, la posologie s'échelonne entre 0,1 et 50 mg par prise et entre 0,1 et 150 mg par jour.

D'une manière préférée, les compositions pharmaceutiques selon l'invention renferment entre 0,1 et 25 mg par prise unitaire, de principe actif de formule générale I ou un de ses sels.

L'invention comprend aussi un procédé pour préparer les composés de formule générale I caractérisé en ce que l'on soumet une cyanoguanidine de formule générale II

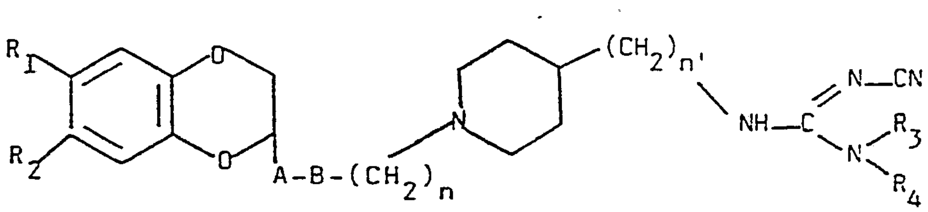

(II)

dans laquelle la définition des substituants R1, R2, R3, R4, A, B, n et n' demeure celle fournie précédemment à l'action d'un agent d'hydrolyse acide pour obtenir la carboxamidoguanidine correspondante de formule générale I

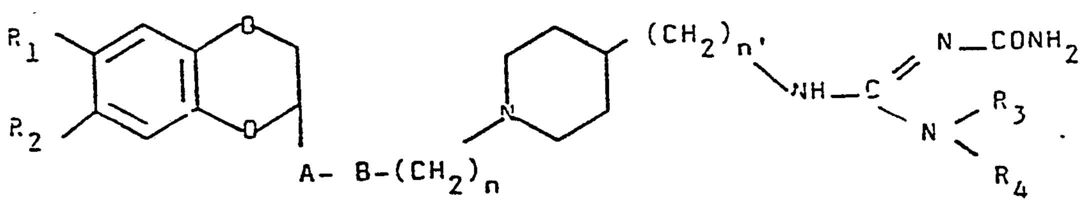

(I)

dans laquelle les substituants R1, R2, R3, R4, A, B, n et n' sont définis comme précédemment que l'on peut si désiré

salifier par addition d'un acide minéral ou organique

dédoubler en isomères optiques par salification à l'aide d'un acide optiquement actif

ou lorsque R4 est de l'hydrogène acyle par action d'un dérivé fonctionnel d'un acide carboxylique pour obtenir un dérivé de formule générale I dans laquelle R4 est un reste acyle.

Selon un mode d'exécution préféré du procédé selon l'invention, l'hydrolyse du groupe cyano est effectuée à température ordinaire au moyen d'un acide minéral concentré comme l'acide sulfurique ou par chauffage en présence d'un acide halohydrique comme l'acide chlorhydrique ou l'acide perchlorique. L'emploi de résines du type polycarboxylique ou polysulfonique peut également être envisagé, comme celle commercialisée sous la marque Nalfion.

Le dédoublement des composés de formule générale I peut être effectué par salification à l'aide d'un acide optiquement actif comme l'acide d-tartrique, l'acide d-dibenzoyltartrique, l'acide d-diéthyltartramique, l'acide pimarique, l'acide abiétique, l'acide d-camphosulfonique, l'acide naphtalène dioxyphosphonique optiquement actif ou l'acide glucose l-phosphorique.

L'acylation des composés de formule générale I dans laquelle R4 est de l'hydrogène peut être réalisée à l'aide d'un dérivé fonctionnel d'acide carboxylique comme par exemple un chlorure d'acide, un anhydride d'acide, un anhydride mixte d'acide formé par réaction avec une dialcoyl — ou dicycloalcoyl carbodiimide en présence ou en l'absence d'un agent favorisant l'acylation comme l'hydroxybenzotriazole, le carbonyl diimidazole, la 4-diméthyl aminopyridine, la pyridine ou l'hexaméthyl phosphorotriamide.

Les cyanoguanidines de départ répondant à la formule générale II sont préparées selon un procédé qui consiste en ce que l'on fait réagir une 4-aminopipéridine de formule générale III

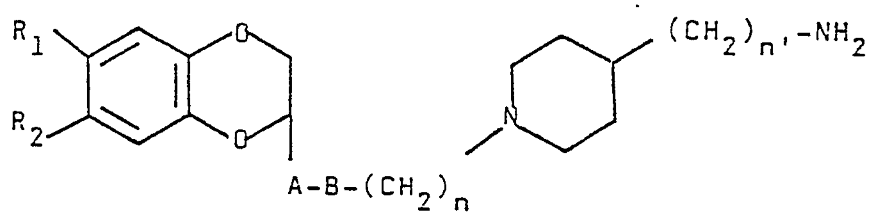

(III)

dans laquelle les substituants A, B, R1, R2, n et n' sont définis comme précédemment avec un réactif de cyano imination choisi dans le groupe constitué parmi les cyanoimino isodithiocarbonates d'alcoyle de formule générale IV

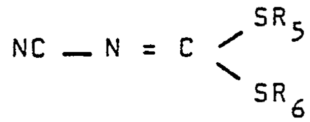

(IV)

dans laquelle R5 et R6 sont des radicaux alcoyle inférieur, et les cyanoimino isothiocarbonates mixtes d'alcoyle de formule générale V

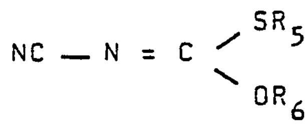

(V)

dans laquelle R5 et R6 sont définis comme précédemment pour former l'isothiourée ou l'isourée de formule générale VI

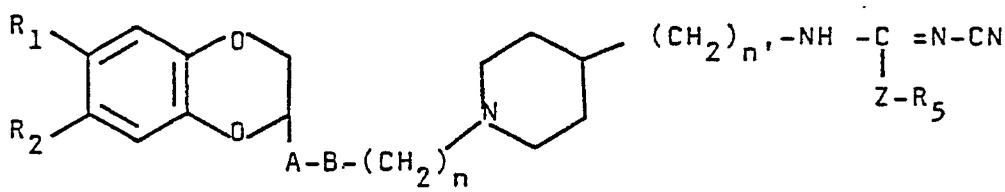

(VI)

dans laquelle les substituants A, B, R1, R2, R5, n et n' sont définis comme précédemment et Z est de l'oxygène ou du soufre, que l'on fait réagir avec une amine primaire ou secondaire de formule R3 NH R4 pour obtenir la cyanoguanidine de formule générale II

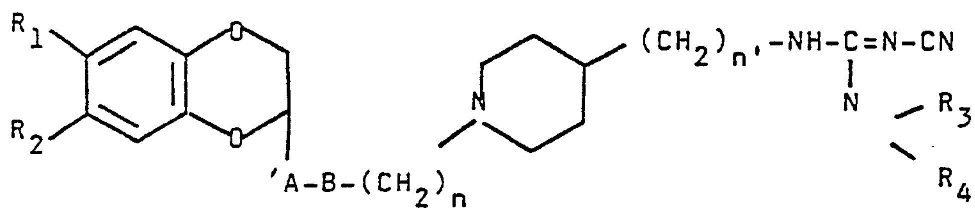

(II)

Les exemples suivants illustrent l'invention.

Exemple I

1-[2,3 dihydro [4H] (1,4 benzodioxin-2yl) méthyl]
4-[(N-carboxamido N'-méthyl) guanidinyl] pipéridine

10 grammes de 1-[2,3 dihydro [4H] (1,4 benzodioxin-2yl) méthyl] 4-[(N-cyano N'-méthyl) guanidinyl] pipéridine sont mis en solution dans 500 ml d'acide sulfurique normal et 50 ml d'éthanol.

Après une semaine de repos à la température ordinaire, la solution est alcalinisée par addition de soude, ce qui provoque la précipitation de cristaux blancs qui sont filtrés, lavés à l'eau et séchés.

Après recristallisation dans l'acétate d'éthyle, on obtient 6,1 grammes de carboxamide cherché sous forme d'écailles blanches. Fi = 94.95°.

Exemple II

1-[2,3 dihydro [4H] (1,4 benzodioxin-2yl) méthyl]
4-[(N-carboxamido N'-allyl) guanidinyl] pipéridine et son fumarate

6 grammes de 1-[2,3 dihydro [4H] (1,4 benzodioxin-2yl) méthyl] 4-[(N-cyano N'-allyl) guanidinyl]

pipéridine sont mis en solution dans 300 ml d'acide sulfurique normal et 25 ml d'éthanol.

Après un repos d'une semaine à la température ambiante, la solution est rendue alcaline par addition de soude et extraite au chloroforme.

La solution chloroformique est lavée à l'eau, séchée et concentrée ce qui permet d'obtenir 6,1 grammes d'un produit huileux que l'on transforme en fumarate par reprise par une solution de 3,3 grammes d'acide fumarique dans 100 ml d'éthanol.

Les cristaux formés sont lavés à l'éthanol et séchés.

On obtient 6,5 grammes de carboxamide cherché sous forme de fumarate. Fi = 200°.

Exemple III

1-[(2,3 dihydro [4H] 1,4 benzodioxin-2yl) méthyl]
4-[(N-carboxamido N' méthyl) guanidinylméthyl] pipéridine et son fumarate

On fait une solution de 3,2 grammes de 1-(2,3 dihydro [4H]-1,4 benzodioxin-2yl méthyl) 4-[(N-cyano N'-méthyl guanidinyl) méthyl] pipéridine dans 100 ml d'une solution d'acide sulfurique normale et laisse reposer une semaine à température ambiante.

La solution est ensuite rendue basique par de la soude, puis extraite au chloroforme et concentrée ce qui donne 3,1 grammes d'huile épaisse.

Le produit ne cristallisant pas, on forme le fumarate par reprise par une solution de 4 grammes d'acide fumarique dans l'éthanol à 95 %.

On obtient 3 grammes de cristaux blancs de PFi = 200-201°.

Le produit cristallise avec 1/2 molécule d'eau.

Exemple IV

1-[(2,3 dihydro [4H] (1,4 benzodioxin-2yl méthyl)
4-[(N-carboxamido N'-triméthyl 1,2,2 propyl) guanidinylméthyl] pipéridine

On met 10 grammes de 1-(2,3 dihydro [4H] 1,4 benzodioxin 2yl méthyl) 4-[(N-cyano N'-triméthyl-1,2,2 propyl) guanidinyl-méthyl] pipéridine en solution dans 200 ml d'une solution d'acide sulfurique normale et 100 ml d'éthanol.

Après une semaine à température ambiante, on rend le milieu réactionnel basique par addition de soude puis extrait la phase aqueuse au chloroforme et concentre à sec la solution chloroformique.

L'huile obtenue est dissoute dans l'acétonitrile d'où le produit cristallise peu après.

Les cristaux obtenus sont filtrés, lavés à l'acétonitrile et séchés.

On obtient 3,5 grammes du dérivé carboxamido cherché sous forme de cristaux de PFi = 164-165°.

Exemple V

1-[(2,3 dihydro [4H] (1,4 benzodioxin-2yl méthyl]
4-[(N-carboxamido N'-allyl) guanidinylméthyl] pipéridine

On met 6 grammes de [2,3 dihydro [4H] (1,4 benzodioxin-2-yl) méthyl]-1 [(N-cyano N'-allyl) guanidinylméthyl]-4 pipéridine en solution dans 150 ml d'une solution normale d'acide sulfurique et on laisse reposer le mélange réactionnel une semaine à la température ambiante.

Après alcalinisation par addition de soude puis extraction au chloroforme, on obtient après évaporation de la solution chloroformique un produit huileux qui est purifié par chromatographie sur une colonne de silice H (Merck) en utilisant le mélange CHC13-isopropylamine comme éluant.

On obtient aussi 4 grammes d'une huile épaisse qui cristallise par traitement à l'éther isopropylique. Le produit pur fond à 80-82°.

Exemple VI

1-(2,3 dihydro [4H] (1,4-benzodioxin-2yl) méthyl 4-[(N-carboxamido
N'-cyclopropyl) guanidinylméthyl] pipéridine.

On met 3,70 g de 1-[2,3 dihydro 4H (1,4 benzodioxin-2yl) méthyl] 4-[(N-cyano N'-cyclopropyl) guanidinylméthyl] pipéridine en suspension dans 50 ml d'acide chlorhydrique à 10 %. On porte au reflux pendant 2 heures puis on laisse revenir la solution réactionnelle à température ambiante. On neutralise par addition de soude puis on épuise la solution par du chloroforme. Les phases chloroformiques sont séparées, lavées à l'eau, séchées sur sulfate de sodium et évaporées à sec.

Le résidu huileux est repris par 50 ml d'une solution de 3 g d'acide fumarique dans l'éthanol. On amorce la cristallisation par grattage puis après repos une nuit à 0° on sépare les cristaux de fumarate. On les rince à l'éthanol puis les sèche sous vide. On obtient ainsi 3,65 g de fumarate de 1-[2,3 dihydro [4H]

(1,4 benzodioxinyl-2) méthyl] 4-[(N carboxamido N'-cyclopropyl guanidinyl) méthyl] pipéridine fondant à 194-196°.

Exemple VII

1 [2,3-dihydro 4H (1,4 benzodioxin-2yl) méthyl]
4-[(N-(carboxamido N'-morpholinyl) guanidinyl) méthyl] pipéridine.

On dissout 4 g de 1-[2,3 dihydro [4H] (1,4-benzodioxin-2yl méthyl] 4-[(N-cyano N'-morpholinyl guanidinyl) méthyl] pipéridine dans 50 ml d'éthanol et on ajoute 10 ml d'acide sulfurique 1N. On laisse le mélange en contact pendant une semaine à température ordinaire puis on neutralise le milieu par addition de soude. La solution est alors épuisée au chloroforme. Les phases chloroformiques sont séparées, lavées à l'eau jusqu'à neutralité, séchées et évaporées à sec. On obtient ainsi 2,80 g de dérivé carboxamido cherché que l'on purifie par recristallisation dans l'éther isopropylique par chaud et froid. Après repos une nuit on sépare les cristaux, les rince à l'éther isopropylique et les sèche sous vide. Le composé pur fond à 150°.

Exemple VIII

De la même façon qu'à l'exemple I les composés suivants ont été préparés :

la 1-[(2,3-dihydro 6-méthyl [4H] 1,4 benzodioxin-2yl méthyl)] 4-[(N-carboxamido N'-allyl) guanidinylméthyl] pipéridine isolée sous forme de fumarate F = 160° environ.

la 1-[(2,3-dihydro [4H] 1,4 benzodioxin-2yl) éthyl] 4-[(N-carboxamido N'-allyl) guanidinyl méthyl] pipéridine isolée sous forme de fumarate F = 230°.

la 1-[(2,3-dihydro [4H] 1,4 benzodioxin-2yl) 2-hydroxyéthyl] 4-[(N-carboxamido N'-méthyl) guanidinylméthyl] pipéridine isolée sous forme de fumarate F = 250°.

la 1-[2,3-dihydro [4H] 1,4 benzodioxin-2yl) éthyl] 4-[(N-carboxamido N'-méthyl) guanidinylméthyl] pipéridine isolée sous forme de fumarate qui cristallise avec une demi-molécule d'eau F = 250°.

Exemple IX

Exemple de réalisation d'une forme pharmaceutique

Comprimés à 5 mg de fumarate de 1-[2,3-dihydro [4H] (1,4-benzodioxin-2yl) méthyl] 4-[(N-carboxamido N'-méthyl) guanidinyl méthyl] pipéridine.

Fumarate de 1-[2,3-dihydro [4H] (1,4 benzodioxin-2yl) méthyl] 4-[(N-carboxamido N'-méthyl) guanidinyl méthyl] pipéridine

| | |
|---|---|
| Amidon de Maïs | 5 g |
| Amidon de Blé | 60 g |
| Gélatine | 80 g |
| Cellulose Microcristalline vendue | 7,5 g |
| sous la marque AVICEL | 35 g |
| Stéarate de Magnésium | 2,5 g |
| Talc | 7,5 g |
| pour 1 000 comprimés | |

Exemple X

Etude pharmacologique des composés selon l'invention.

a) Détermination de la toxicité aiguë

Une dose léthale 50 % (DL50) approchée a été déterminée, après administration par voie orale des composés selon l'invention, sur des lots de 8 souris femelles EOPS d'élevage CESAL par la méthode de D.E.J. Campbell et W. Richter (Acta Pharmacol. and Toxicol. 25 (1967) 345).

Les animaux ont été gardés en observation pendant 5 jours. Les doses léthales moyennes des produits selon l'invention s'échelonnent entre 600 et 880 mg/kg.

b) Recherche d'un effet sur le système nerveux central

Les composés selon l'invention ont été administrés par voie orale à des lots de 5 souris femelles EOPS d'élevage CESAL à des doses variant de 5 à 50 mg/kg.

Les tests tels que traction, cheminée, évasion, planche à trous, température, ptosis, électrochocs ont été effectués une heure après gavage.

Les composés selon l'invention à la dose de 50 mg/kg déterminent une légère ptose des paupières, un abaissement de la température, une diminution de la curiosité et de la mobilité.

c) Détermination des effets sur la tension artérielle du Rat

L'essai a été effectué sur des rats normo-tendus préalablement anesthésiés auxquels les produits selon l'invention sont administrés par voie intraveineuse à des doses allant de 0,2 mg à 2 mg/kg. Ces doses provoquent une très nette hypotension, une bradycardie légère, et une nette inhibition adrénergique.

**Revendications** (pour les Etats contractants : BE, CH, DE, FR, GB, LI, LU, NL, SE)

1. Les carboxamidoguanidines choisies dans le groupe constitué par :

a) Les carboxamidoguanidines de formule générale I

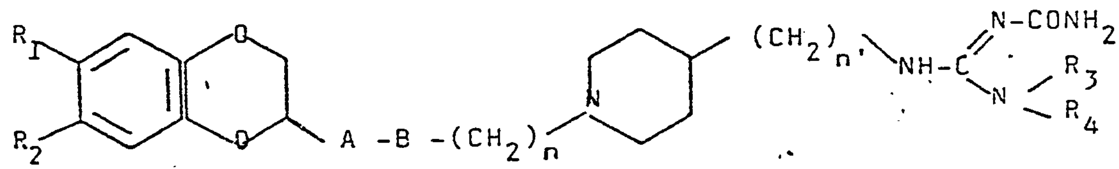 (I)

dans laquelle

$R_1$ et $R_2$, distinctement l'un de l'autre, représentent de l'hydrogène, un halogène, un radical alcoyle ayant de 1 à 6 atomes de Carbone, un radical alcoxy inférieur dont la partie alcoyle comporte de 1 à 6 atomes de Carbone ou un radical trifluorométhyle.

$R_3$ représente un radical alcoyle inférieur ayant de 1 à 6 atomes de Carbone, un radical cyclopropyle ou un radical allyle.

$R_4$ représente de l'hydrogène, un radical alcoyle ayant de 1 à 6 atomes de Carbone ou un reste acyle dérivé d'un acide carboxylique ayant de 1 à 12 atomes de Carbone,

ou bien $R_3$ et $R_4$ forment ensemble le reste alcoylène d'un hétérocycle azoté ayant de 3 à 7 maillons pouvant comporter un second hétéroatome, choisi dans le groupe constitué par l'azétidine, l'aziridine, la pyrolidine, la pipéridine, l'hexahydropyrimidine, la tétrahydrothiazine, l'hexaméthylène imine, l'heptaméthylène imine, la morpholine, la pipérazine, une alcoylpipérazine, une (hydroxyalcoyl) pipérazine et une (acyloxyalcoyl) pipérazine

n représente 0 ou 1

n' représente 0 ou 1

A représente une liaison simple ou $—CH_2$ et B représente —CHOH— ou C = 0 ou A représente —CHOH— ou C = 0 et B représente une liaison simple ou $—CH_2—$

b) Les carboxamidoguanidines de formule générale $I_A$

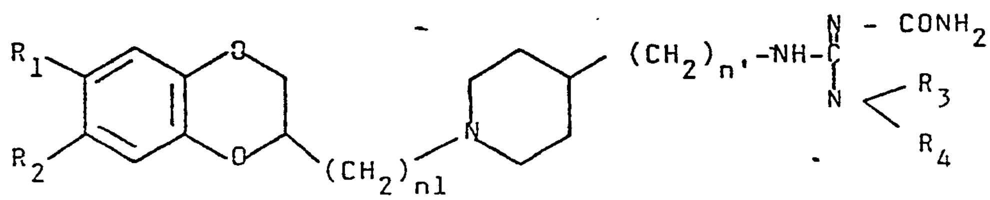 (IA)

dans laquelle $n_1$ représente un nombre entier variant de 0 à 3 et les substituants $R_1$, $R_2$, $R_3$, $R_4$ et n' ont les significations fournies précédemment.

2. Les sels d'addition des composés de formule générale I avec un acide minéral ou organique, de préférence un acide thérapeutiquement compatible selon la revendication 1.

3. Les isomères optiquement actifs des composés de formule générale I et les isomères syn et anti des composés de formule générale I, selon la revendication 1.

4. Les carboxamidoguanidines selon l'une des revendications 1 à 3 choisies dans le groupe constitué par

a) Les composés de formule générale $I_A$

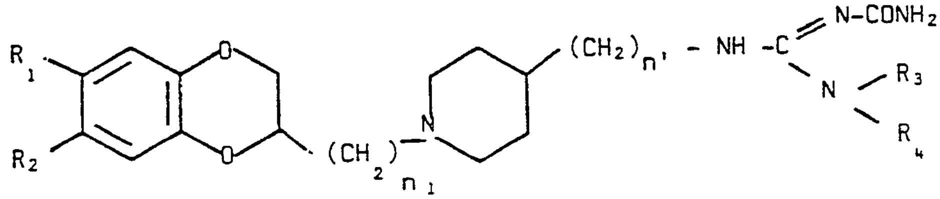 (IA)

dans laquelle $n_1$ représente un nombre entier variant de 0 à 3 et les substituants $R_1$, $R_2$, $R_3$, $R_4$ et n' ont les significations mentionnées à la revendication 1 et

b) les sels d'addition de ces composés avec un acide minéral ou organique

5. Les composés selon l'une des revendications 1 à 3 de formule IB

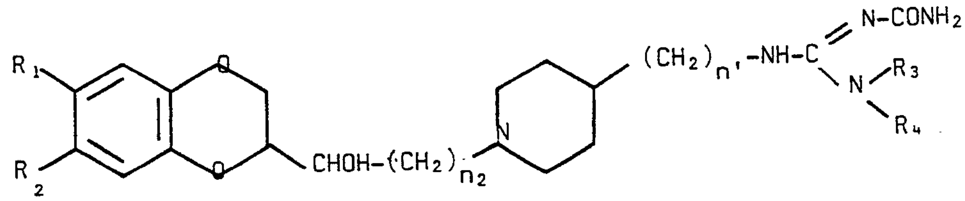

(IB)

dans laquelle $n_2$ représente un nombre entier variant de 0 à 2 et les substituants $R_1$, $R_2$, $R_3$, $R_4$ et n' ont les définitions mentionnées à la revendication 1, ainsi que les sels d'additions de ces composés avec un acide minéral ou organique.

6. Les composés selon l'une des revendications 1 à 3 de formule IC

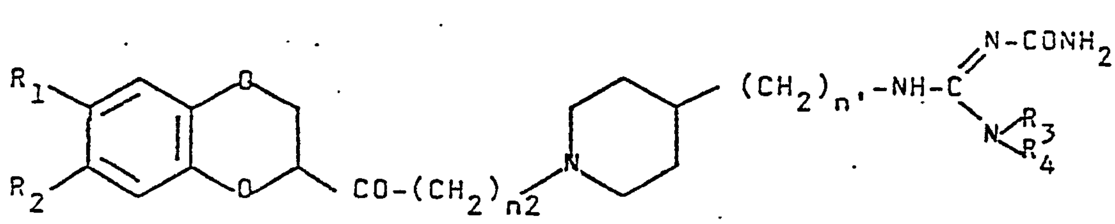

(IC)

dans laquelle R1, R2, R3, R4, n' et n2 ont les significations mentionnées dans la revendication 1 ainsi que leurs sels d'addition avec un acide minéral ou organique.

7. Un composé selon l'une des revendications 1 à 4 à savoir la 1-[2,3 dihydro [4H] (1,4-benzodioxin-2yl) méthyl] 4-(N-carboxamido N'-méthyl guanidinyl) pipéridine.

8. Un composé selon l'une des revendications 1 à 4 à savoir la 1-[2,3-dihydro [4H] (1,4-benzodioxin-2yl) méthyl] 4-[(N-carboxamido N'-méthyl guanidinyl) méthyl] pipéridine.

9. Un composé selon l'une des revendications 1 à 4 à savoir la 1-[(2,3-dihydro [4H] (1,4-benzodioxin-2yl) méthyl] 4-[(N-carboxamido N'-allyl guanidinyl) méthyl] pipéridine.

10. Un composé selon l'une des revendications 1 à 4 à savoir la 1-[(2,3-dihydro [4H] 1,4-benzodioxin-2yl) éthyl] 4-[(N-carboxamido N'-allyl guanidinyl) méthyl] pipéridine.

**Revendications** (pour l'Etat contractant AT)

1. Un procédé pour préparer les composés de formule générale I

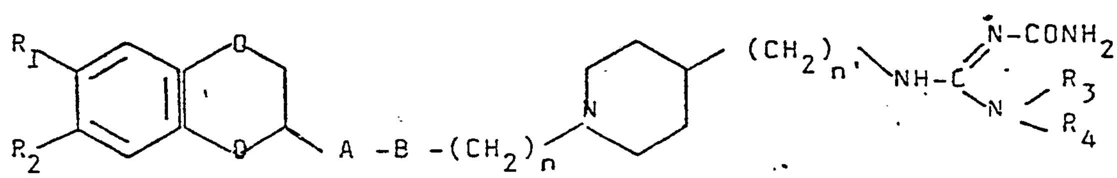

(I)

dans laquelle

$R_1$ et $R_2$ distinctement l'un de l'autre représentent de l'hydrogène, un radical alcoyle ayant de 1 à 6 atomes de Carbone ; un radical alcoxy inférieur dont la partie alcoyle comporte de 1 à 6 atomes de Carbone, un halogène ou un radical trifluorométhyle.

$R_3$ est un radical alcoyle inférieur ayant de 1 à 6 atomes de Carbone, un radical cyclopropyle ou un radical allyle,

$R_4$ représente l'hydrogène, un radical alcoyle ayant 1 à 6 atomes de carbone, ou un reste acyle dérivé d'un acide carboxylique ayant de 1 à 12 atomes de Carbone,

ou bien $R_3$ et $R_4$ forment ensemble le radical alcoylène d'un hétérocycle azoté ayant de 3 à 7 maillons éventuellement interrompus par un second hétéroatome choisi dans le groupe constitué par l'azétidine, l'aziridine, la pyrolidine, la pipéridine, l'hexahydropyrimidine, la tétrahydrothiazine, l'hexaméthylène imine, l'heptaméthylène imine, la morpholine, la pipérazine, une alcoylpipérazine, une (hydroxyalcoyl) pipérazine et une (acyloxyalcoyl) pipérazine.

n représente 0 ou 1

n' représente 0 ou 1

A représente une liaison simple ou —$CH_2$— et B représente —CHOH— ou C = 0 ou A représente —CHOH— ou C = 0 et B représente une liaison simple ou —$CH_2$—
caractérisé en ce que l'on soumet une cyanoguanidine de formule générale II

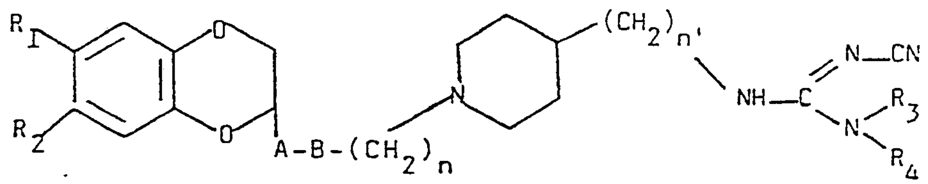

(II)

dans laquelle la définition des substituants $R_1$, $R_2$, $R_3$, $R_4$, A, B, n et n' demeure celle fournie précédemment à l'action d'un agent d'hydrolyse acide pour obtenir la carboxamidoguanidine correspondante de formule générale I

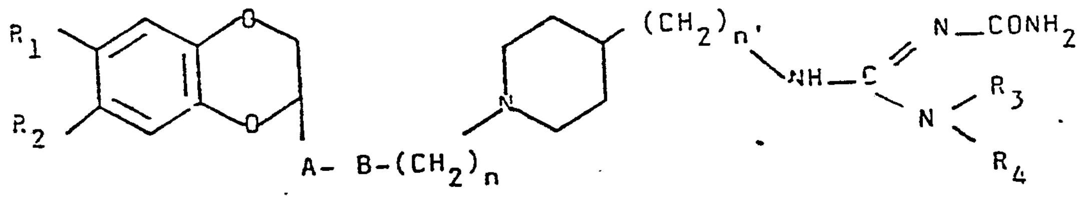

(I)

dans laquelle les substituants $R_1$, $R_2$, $R_3$, $R_4$, A, B, n et n' sont définis comme précédemment que l'on peut si désiré

salifier par addition d'un acide minéral ou organique ou dédoubler en ses isomères optiques par salification à l'aide d'un acide optiquement actif

ou lorsque $R_4$ est de l'hydrogène, acyler par action d'un dérivé fonctionnel, d'un acide carboxylique pour obtenir un dérivé de formule générale I dans laquelle $R_4$ est un reste acyle.

2. Un procédé selon la revendication 1 pour préparer la 1-[2,3-dihydro [4H] (1,4-benzodioxin-2yl) méthyl] 4-(N-carboxamido N'-méthyl) guanidinyl] pipéridine caractérisé en ce qu'on soumet la 1-[2,3-dihydro [4H] 1,4-benzodioxin-2yl) méthyl 4-[N-cyano N'-méthyl) guanidinyl] pipéridine à une hydrolyse acide.

3. Un procédé selon la revendication 1 pour préparer la 1-[2,3-dihydro [4H] (1,4-benzodioxin-2yl) méthyl] 4-[(N-carboxamido N'-allyl) guanidinyl] pipéridine caractérisé en ce qu'on soumet la 1-[2,3-dihydro [4H] (1,4-benzodioxin-2yl) méthyl] 4-[(cyano N'-allyl) guanidinyl] pipéridine à une hydrolyse acide.

4. Un procédé selon la revendication 1 pour préparer la 1-[(2,3-dihydro [4H] 1,4-benzodioxin-2yl) méthyl] 4-[(N-carboxamido N'-méthyl) guanidinyl méthyl] pipéridine caractérisé en ce qu'on soumet la 1-[(2,3-dihydro [4H] 1,4 benzodioxin-2yl) méthyl] 4-[(N-cyano N'-méthyl) guanidinylméthyl] pipéridine à une hydrolyse acide.

5. Un procédé selon la revendication 1 pour préparer la 1-[(2,3-dihydro [4H] 1,4-benzodioxin-2yl) méthyl] 4-[N-carboxamido N'-(1,2,2-triméthylpropyl) guanidinylméthyl] pipéridine caractérisé en ce qu'on soumet la 1-[(2,3-dihydro [4H] 1,4-benzodioxin-2yl) méthyl] 4-[N-cyano N'-(1,2,2-triméthylpropyl) guanidinylméthyl] pipéridine à une hydrolyse acide.

6. Un procédé selon la revendication 1 pour préparer la 1-[(2,3-dihydro [4H] 1,4-benzodioxin-2yl) méthyl] 4-[(N-carboxamido N'-cyclopropyl) guanidinyl méthyl] pipéradine caractérisé en ce qu'on soumet la 1-[(2,3-dihydro [4H] 1,4-benzodioxin-2yl) méthyl] 4-[(N-cyano N'-cyclopropyl) guanidinyl méthyl] pipéridine à une hydrolyse acide.

7. Un procédé selon la revendication 1 pour préparer la 1-[(2,3-dihydro [4H] 1,4-benzodioxin-2yl) méthyl] 4-[(N-carboxamido N'-morpholinyl) guanidinylméthyl] pipéradine caractérisé en ce qu'on soumet la 1-[(2,3-dihydro [4H] 1,4-benzodioxin-2yl) méthyl] 4-[(N-cyano N'-morpholinyl) guanidinyl méthyl] pipéridine à une hydrolyse acide.

**Claims** (for the Contracting States : BE, CH, LI, DE, FR, GB, LU, NL, SE)

1. The carboxamido guanidines selected from the group consisting of
   a) The carboxamidoguanidines of general formula I

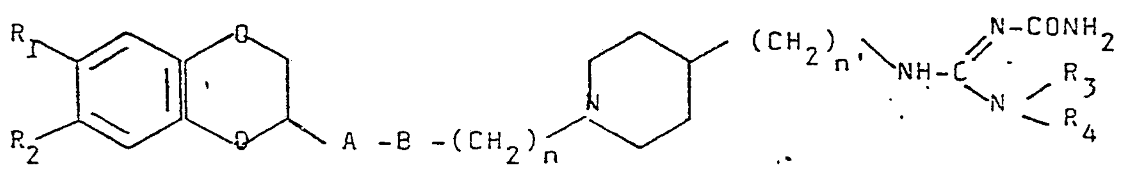

(I)

wherein

$R^1$ and $R^2$ different each other, are a hydrogen, a halogen, a lower alkyl radical having from 1 to 6 carbon atoms, a lower alkoxy radical the alkyl moiety of which includes from 1 to 6 carbon atoms or a trifluoromethyl radical.

$R^3$ is a lower alkyl radical having from 1 to 6 carbon atoms, a cyclopropyl rádical or an allyl radical.

$R^4$ is a hydrogen, an alkyl radical having from 1 to 6 carbon atoms or the acyl residue of a carboxylic acid having from 1 to 12 carbon atoms or $R^3$ and $R^4$ together are the alkylene residue of a nitrogen-bearing heteroring having from 3 to 7 links which may include an extra heteroatom, selected from the group consisting of azetidine, aziridine, pyrolidine, pipéridine, hexahydropyrimidine, tetrahydrothiazine, hexamethylene imine, heptamethylene imine, morpholine, piperazine, an alkyl piperazine, a (hydroxy alkyl) piperazine and an (acyloxyalkyl) piperazine

n is 0 or 1

n′ is 0 or 1

A is a direct bond or $—CH^2—$ and B is CHOH or $>$ C = 0 or A is CHOH or $>$ C = 0 and B is a direct bond or $—CH^2—$ and

b) The carboxamidoguanidines of general formula $I_A$

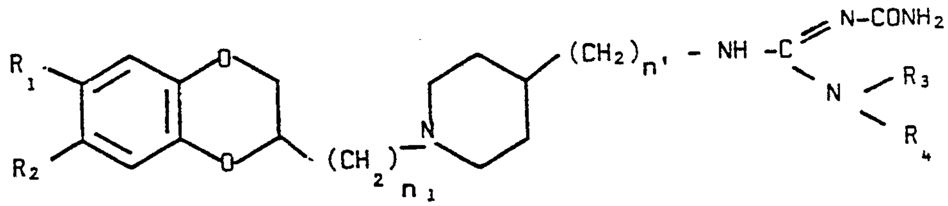

(IA)

wherein $n^1$ is an integer from 0 to 3 and the substituents $R^1$, $R^2$, $R^3$, $R^4$ and n′ have the above — given definitions.

2. The addition salts of the compounds of general formula I with a mineral or organic acid, preferably a therapeutically-compatible acid, according to claim 1.

3. The optically-active isomers of the compounds of general formula I and the isomers syn and anti of the compounds of general formula I according to claim 1.

4. The carboxamidoguanidines according to any of claims 1 to 3 selected from the group consisting of

a) The compounds of general formula $I_A$

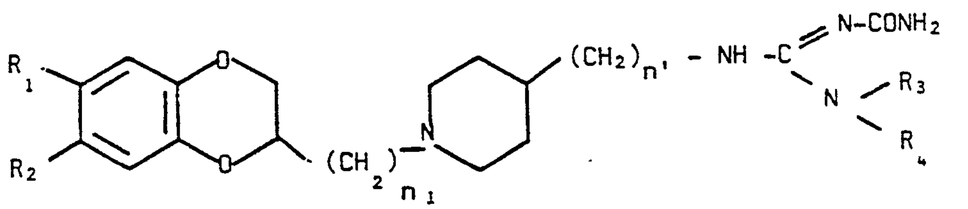

(IA)

wherein $n^1$ is an integer from 0 to 3 and the substituents $R^1$, $R^2$, $R^3$, $R^4$ and n′ have the meanings as defined in claim I and

b) The acid addition salts thereof with a mineral or organic acid

5. The compounds according to any of claims 1 to 3 having the formula $I_B$

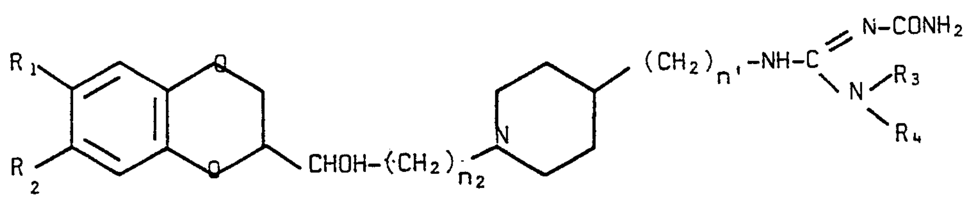

(IB)

wherein $n^2$ is an integer from 0 to 2 and the substituents $R^1$, $R^2$, $R^3$, $R^4$, and n′ having the same meaning as defined in claim 1 as well as the acid addition salts thereof with a mineral or organic acid.

6. The compounds according to any of the claim 1 to 3 having the formula $I_C$

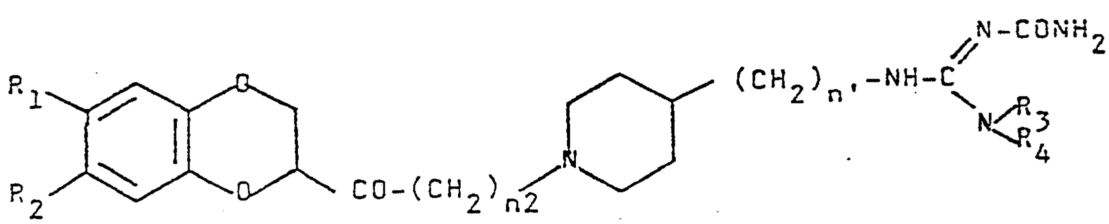

(IC)

wherein $R^1$, $R^2$, $R^3$, $R^4$, n' and $n^2$ have the meanings given in claim 1 as well as the addition salts thereof with a mineral or organic acid.

7. A compound according to any of claim 1 to 4 i. e. 1-[2,3-dihydro [4] (1,4-benzodioxin-2yl) methyl] 4-N-carboxamido N'-methylguanidinyl) piperidine.

8. A compound according to any of the claims 1 to 4 i. e. 1-[2,3-dihydro [4H] (1,4-benzodioxin-2yl) methyl] 4-[(N-carboxamido N'-methyl guanidinyl) methyl] piperidine.

9. A compound according to any of the claims 1 to 4 i. e. 1-[(2,3-dihydro [4H] (1,4-benzodioxin-2yl) methyl] 4-[(N-carboxamido N'-allyl guanidinyl) methyl] piperidine.

10. A compound according to any of the claims 1 to 4 i. e. 1-[(2,3-dihydro [4H] 1,4-benzodioxin-2yl) methyl] 4-(N-carboxamido N'-allyl guanidinyl) methyl] piperidine.

**Claims** (for the Contracting State AT)

1. A process for producing the compounds of general formula I

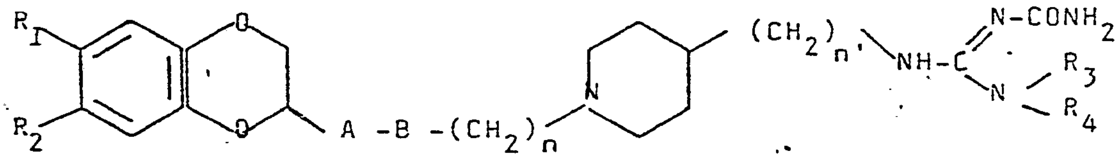

(I)

wherein

$R^1$ and $R^2$, distinctly each other, are a hydrogen, an alkyl radical having from 1 to 6 carbon atoms, a lower alkoxy radical, the alkyl moiety of which includes from 1 to 6 carbon atoms, a halogen or a trifluoromethyl radical.

$R^3$ is a lower alkyl radical having from 1 to 6 carbon atoms, a cyclopropyl radical, an allyl radical or the acyl residue of a carboxylic acid having from 1 to 12 carbon atoms.

$R^4$ is a hydrogen or an alkyl radical having from 1 to 6 carbon atoms

or $R^3$ and $R^4$ together are the alkylene residue of a nitrogen-bearing hetero-ring having from 3 to 7 links optionally interrupted by a further heteroatom, selected from the group consisting of azetidine, aziridine, pyrolidine, piperidine, hexahydro pyrimidine, tetrahydrothiazine, hexamethylene imine, heptamethylene imine, morpholine, piperazine, alkylpiperazine, hydroxyalkyl piperazine and (acyloxy alkyl) piperazine.

n is zero or 1

n' is zero or 1

A is direct bond or $—CH^2—$ and B is —CHOH— or > C = 0 or A is CHOH or ⁀ C = 0 and B is a direct bond or $—CH^2—$

characterised in that a cyanoguanidine of general formula II

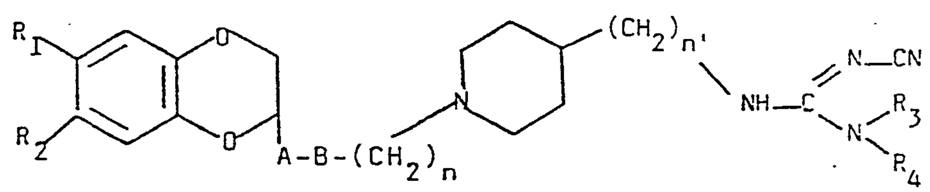

(II)

wherein the meanings the substituents $R^1$, $R^2$, $R^3$, $R^4$, A, B, n and n' remain the same as those previously-given is submitted to the action of a hydrolysing agent to produce the corresponding carboxamidoguanidine of general formula I

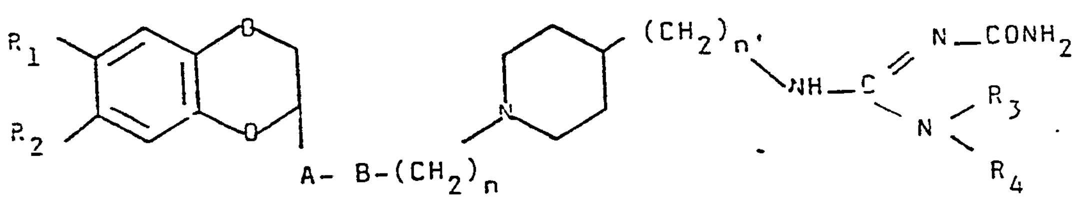

(I)

in which the definitions of the substituents $R^1$, $R^2$, $R^3$, $R^4$, A, B, n and n' are the same as previously-given which may, when desired, be

salified by adding a mineral or organic acid

resolved into its optical isomers by salifying it with an optically-active acid or when $R^4$ is hydrogen, acylated using a functional derivative of a carboxylic acid to produce a compound of general formula I in which $R^4$ is an acyl moiety.

**Patentansprüche** (für die Vertragsstaaten : BE, CH, LI, DE, FR, GB, LU, NL, SE)

1. Die Carboxamidoguanidine aus Die Gruppe von

a) die Carboxamidoguanidine der allgemeinen Formel I

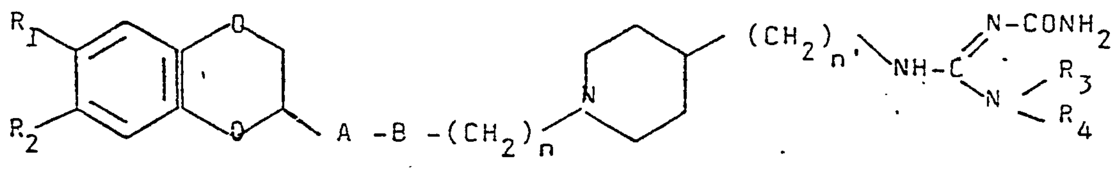

(I)

worin

$R^1$ und $R^2$, verschieden einander, Wasserstoff, Halogen, ein Alkyl radikal mit 1 bis 6 Kohlenstoffatomen, ein niedrig-alkoxy Radikal dessen Alkyl Teil 1 bis 6 Kohlenstoff-atomen enthält oder ein Trifluoromethyl Radikal bedeuten

$R^3$ ein niedrig-Alkyl Radikal mit 1 bis 6 Kohlenstoff-Atomen, ein Cyclopropyl Radikal oder ein Allyl Radikal bedeutet

$R^4$ Wasserstoff, ein Alkyl Radikal mit 1 bis 6 Kohlenstoffatomen oder ein Acylrest von einer Carbonsäure mit 1 bis 12 Kohlenstoff-atomen darstellt

oder $R^3$ und $R^4$ zusammen, den Alkylenrest eines Stickstoff-haltige Heteroring der eine zweite Heteroatom enthalten kann, aus der Gruppe gewählt die Azetidin, Aziridin, Pyrolidin, Piperidin, Hexahydropyrimidin, Tetrahydrothiazin, Hexamethylenimin, Heptamethylen imin, Morpholin, Piperazin, (Alkylpiperazin), (Hydroxyalkyl piperazin), und (Acyloxyalkyl piperazin) enthält

n 0 oder 1 ist

n' 0 oder 1 ist

A eine einfache Bund oder $—CH^2—$ ist und B —CHOH— oder $>C=0$ darstellt oder A CHOH oder $C=0$ bedeutet und B eine einfache Bund oder $—CH^2—$ ist und

b) die Carboxamidoguanidinen der allgemeinen Formel $I_A$

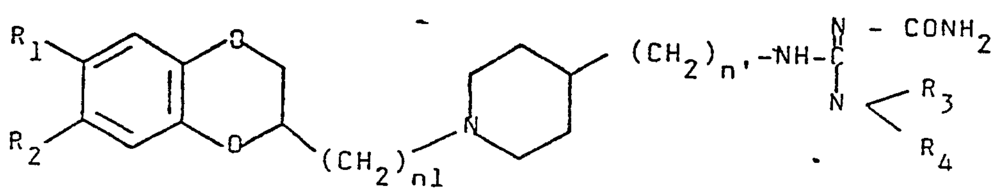

(IA)

in der $n^1$ is ein ganze Zahl von 0 bis 3 und die Substituenten $R^1$, $R^2$, $R^3$, $R^4$ und n' die selbe Bedeutungen als vorher haben.

2. Die Zusatzsalze der Verbindungen der allgemeinen Formel I mit einer anorganischen oder organischen Säure, vorzüglich eine therapeutische-verträglichen Säure, nach Anspruch 1.

3. Die optisch-aktive Isomere der Verbindungen der allgemeinen Formel I und die Syn oder Anti Isomeren der Verbindungen der allgemeinen Formel I nach Anspruch 1.

4. Die Carboxamidoguanidinen nach einem der Ansprüche 1 bis 3 aus die Gruppe gewählt die

a) die Verbindungen der allgemeinen Formel $I_A$

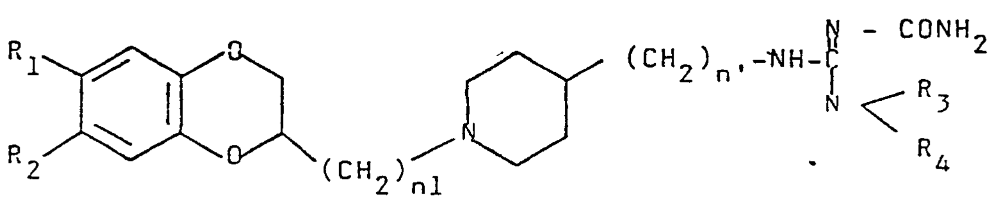

(IA)

worin $n^1$ ein ganze Zahl von 0 bis 3 ist und die Substituenten $R^1$, $R^2$, $R^3$, $R^4$ und n' die in dem Anspruch 1 ansgeführte Bedeutungen haben und

b) die Zusatzsalze dieser Verbindungen mit einer anorganischen oder organischen Säure enthält.

5. Die Verbindungen nach einem der Ansprüche 1 bis 3 der allgemeinen Formel $I_B$

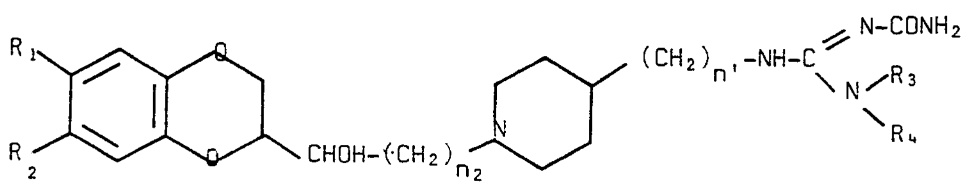

(IB)

worin $n^2$ ein ganze Zahl von 0 bis 2 ist die Substituenten $R^1$, $R^2$, $R^3$, $R^4$ und n' die in dem Anspruch 1 angeführte Bedeutungen haben, sowie als die Zusatzsalze dieser Verbindungen mit einem anorganischen oder organischen Säure.

6. Die Verbindungen nach einem der Ansprüche 1 bis 3, der Formel $I_C$

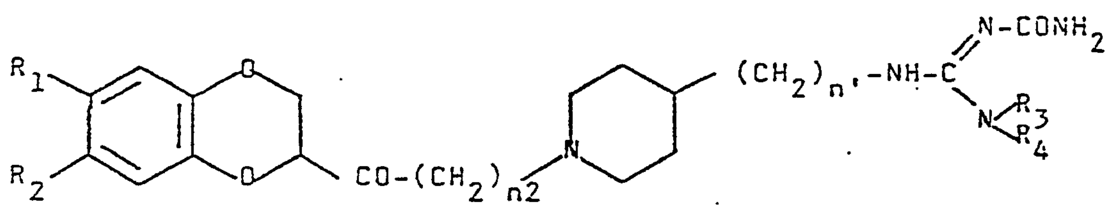

(IC)

worin $R^1$, $R^2$, $R^3$, $R^4$, n' und $n^2$ die in dem Anspruch 1 angeführte Bedeutungen haben, sowie als die Zusatzsalze einer anorganischen oder organischen Säure.

7. Eine Verbindung nach einem der Ansprüche 1 bis 4 i. e. 1 [2,3-dihydro [4H] (1,4-Benzodioxin-2yl) methyl] 4(N-carboxamido N'-methyl guanidinyl) piperidin.

8. Eine Verbindung nach einem der Ansprüche 1 bis 4 i. e. 1 [(2,3-dihydro [4H] 1,4-Benzodioxin-2yl) methyl) [(4N-carboxamido N'-methyl guanidinyl) methyl] piperidin.

9. Eine Verbindung nach einem der Ansprüche 1 bis 4 i. e. 1 [2,3-dihydro [4H] (1,4-Benzodioxin-2yl) methyl] 4-[(N-carboxamido N'-allyl guanidinyl) methyl] piperidin.

10. Eine Verbindung nach einem der Ansprüche 1 bis 4 i. e. 1 [(2,3-dihydro [4H] 1,4-Benzodioxin-2yl) ethyl 4 [(N-carboxamido N'-allyl guanidinyl) methyl] piperidin.

**Patentansprüche** (für den Vertragsstaat AT)

1. Ein Verfahren für die Herstellung der Verbindungen der allgemeinen Formel I

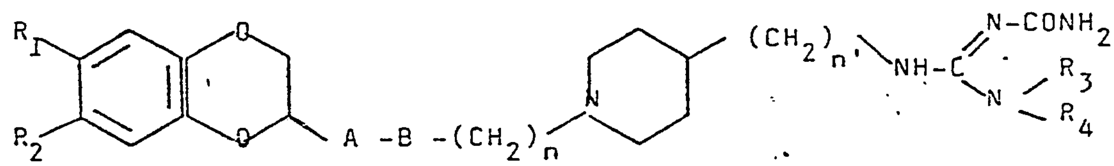

(I)

worin

$R^1$ und $R^2$, verschieden einander, Wasserstoff, Halogen, ein Alkyl radikal mit 1 bis 6 Kohlenstoffatomen, ein niedrig-alkoxy Radikal dessen Alkyl Teil 1 bis 6 Kohlenstoff-atomen enthält oder ein Trifluoromethyl Radikal bedeuten

$R^3$ ein niedrig-Alkyl Radikal mit 1 bis 6 Kohlenstoff-Atomen, ein Cyclopropyl Radikal oder ein Allyl Radikal bedeutet

$R^4$ Wasserstoff, ein Alkyl Radikal mit 1 bis 6 Kohlenstoffatomen oder ein Acylrest von einer Carbonsäure mit 1 bis 12 Kohlenstoff-atomen darstellt

oder $R^3$ und $R^4$ zusammen, den Alkylenrest eines Stickstoff-haltige Heteroring der eine zweite Heteroatom enthält oder aus der Gruppe gewählt die Azetidin, Aziridin, Pyrolidin, Piperidin, Hexahydropyrimidin, Tetrahydrothiazin, Hexamethylenimin, Heptamethylen imin, Morpholin, Piperazin, Alkylpiperazin, (Hydroxyalkyl piperazin), und (Acyloxyalkyl piperazin) enthält

n 0 oder 1 ist

n' 0 oder 1 ist

A eine einfache Bund oder —$CH^2$— ist und B —CHOH— oder $>$C = 0 darstellt oder A CHOH oder C = 0 bedeutet und B eine einfache Bund oder —$CH^2$— ist

dadurch gekennzeichnet dass ein Cyanguanidin der allgemeinen Formel II

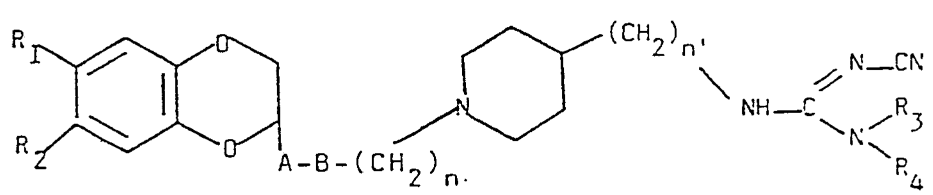

(II)

worin die Bedeutungen der substituenten $R^1$, $R^2$, $R^3$, $R^4$, A, B, n und n' die Salbse als vorherangegeben, bleiben, mit einem säurem Hydrolysismittel ernwirken 6 lässt um die entsprechende Carboxamidoguanidin der allgemeinen Formel I

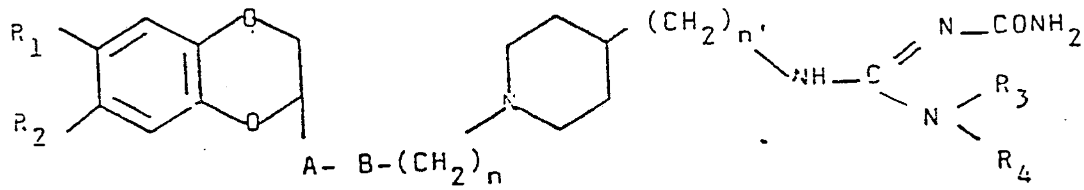

(I)

zu erhalten

die gegebenenfalls

in einem Salz durch Zusatz einer anorganischen oder organischen Säure uberführt oder in seinen optischen Isomeren durch Salzmachung mit einer optisch-aktiven Säure spaltet

oder wenn $R^4$ ein Wasserstoff ist, durch Einwirkung eines funktionnell erivates einer Carbonsäure acyliert lässt um eine Verbindung der allgemeinen Formel I, in der $R^4$ ein Acyl Radikal ist, zu erhalten.

2. Ein Verfahren nach Anspruch 1 für die Herstellung von 1-[2,3-Dihydro [4H] (1,4-Benzodioxin-2yl methyl] 4(N-carboxamido N'-methyl) guanidinyl] piperidin dadurch gekennzeichnet dass man 1-[2,3 Dihydro [4H] benzodioxin-2yl) methyl] 4-[N-cyano N'-methyl) guanidinyl] piperidin eine säure Hydrolysis einwirken lässt.

3. Ein Verfahren nach Anspruch 1 für die Herstellung von 1-[2,3-Dihydro [4H] (1,4 Benzodioxin-2yl) methyl] 4-N-carboxamido N'-allyl) guanidinyl piperidin dadurch gekennzeichnet dass man 1-[2,3-Dihydro [4H] benzodioxin-2-yl) methyl] 4-[(N-cyano N'-allyl guanidinyl] piperidin einer säure Hydrolysis einwirken lässt.

4. Ein Verfahren nach Anspruch 1 für die Herstellung von 1-[2,3-Dihydro [4H] 1,4-benzodioxin-2yl) methyl] 4-[(N-carboxamido N' methyl) guanidinyl methyl] piperidin dadurch gekennzeichnet dass man 1-[(2,3-Dihydro [4H] 1,4-Benzodioxin-2yl) methyl] 4-[(N-cyano N'-methyl) guanidinyl methyl] piperidin eine säure Hydrolysis einwirken lässt.

5. Ein Verfahren nach Anspruch 1 für die Herstellung von 1 [(2,3-Dihydro [4H] 1,4-benzodioxin-2yl) methyl] 4-[(N-carboxamido N'-1,2,2-trimethyl propyl) guanidinyl methyl] piperidin dadurch gekennzeichnet dass man 1-[(2,3-Dihydro [4] 1,4-benzodioxin-2yl) methyl] 4-N-cyano N'-[(1,2,2-trimethyl propyl) guanidinyl-methyl] piperidin eine säure Hydrolysis einwirken lässt.

6. Ein Verfahren nach Anspruch 1 für die Herstellung von 1-[(2,3-Dihydro [4H] 1,4-benzodioxin-2yl) methyl] 4-[(N-carboxamido N'-cyclopropyl) guanidinylmethyl] piperidin dadurch gekennzeichnet dass man 1-[2,3-dihydro [4H] 1,4-benzodioxin-2yl) methyl] 4-[(N-cyano N'-cyclopropyl) guanidinylmethyl)] piperidin eine säure Hydrolysis einwirken lässt.

7. Ein Verfahren nach Anspruch 1 für die Herstellung von 1-[(2,3-Dihydro [4H] 1,4-benzodioxin-2yl) methyl] 4 [(N-carboxamido N' morpholinyl) guanidinyl methyl] piperidin dadurch gekennzeichnet dass man 1-[(2,3-Dihydro [4H] 1,4 benzodioxin-2yl) methyl 4-[(N-cyano N'-morpholinyl) guanidinyl methyl] piperidin eine säure Hydrolysis einwirken lässt.